# EUROPEAN PATENT APPLICATION

(11) **EP 4 706 495 A1**
(43) Date of publication of application: **11.03.2026**
(21) Application number: 25196038.1
(22) Date of filing: 14.08.2025
(51) Int. Cl.: A61B 1/00, A61M 1/00, A61M 39/22

(54) **SUCTION VALVE, FLUID CONTROL UNIT, METHODS FOR MANUFACTURING AND SURGICAL DEVICE**

(30) Priority: 06.09.2024 DE 102024125705
(71) Applicant: Karl Storz SE & Co. KG, 78532 Tuttlingen (DE)
(72) Inventor: POLLUKS, Indrek-Toomas, 78532 Tuttlingen (DE)

(57) **Abstract**

The invention relates to suction valve for controlling the communication of fluid channels of a surgical device, in particular an endoscope, comprising a suction chamber; a piston assembly for controlling fluid flow through the suction chamber, wherein the suction chamber and the piston assembly are configured as a single-piece injection-molded workpiece, wherein the suction chamber is formed by a hollow part of the workpiece, which is pulled over the piston assembly. Furthermore, the invention relates to a fluid control unit and a surgical device, as well as to methods for manufacturing a suction valve and a fluid control unit.

## Description

### FIELD OF THE INVENTION

The present invention relates to a suction valve for controlling the communication of fluid channels of a surgical device, in particular an endoscope, and to a corresponding method for manufacturing a suction valve. The present invention further relates to a fluid control unit for controlling communication of fluid channels of a surgical device, in particular an endoscope, and to a corresponding method for manufacturing a fluid control unit. The present invention further relates to a surgical device.

### BACKGROUND OF THE INVENTION

Surgical devices, such as general, therapeutic or diagnostic endoscopes, resecto-scopes, etc., are utilized among others for visually intracorporeal inspection, diagnosis and surgical assistance.

Surgical devices, such as an endoscope, are often equipped with a fluid mechanism for cleaning and removing filthy matters, body fluids and excised tissues. For example, in order to ensure a minimally invasive surgery, when required, any excess material is flushed out of the deep body cavities or internal organs together with the sucked-up fluids through an opening at a distal tip of an endoscope.

In order to provide such a fluid mechanism, suction valves are engaged in controlling the suction function by connecting or disconnecting the fluid channels that are connectable to the fluid mechanism of the endoscope. These fluid channels can be connected at one end to a distal tip of the endoscope and at the other end to a suction source, e.g. a suction pump, which generates negative pressure.

A suction valve has generally two operating states and can operate by manually switching between these two states by means of a valve button. If the valve button remains in the released state, the suction valve blocks the suction function to the
distal tip of the endoscope. Accordingly, the suction valve is functionally divided into two fluid isolated parts, and therein the fluid channels for the suction function are disconnected and separated by the suction valve. When the valve button is pressed down, the suction channels are in fluid communication, which allows the generated negative pressure to be propagated throughout the suction channels and suck for example body secretions, flushing liquid or the like from the distal tip of the endoscope out of a body cavity. When the valve button is manually released and popped up, the suction valve blocks fluid flow and stops the suction function again.

The above-described conventional suction valve usually comprises multiple individual components, such as a piston, at least two O-rings or seals, a spring, a button, a lower sealing element, a housing, and so on. The assembly of these components is therefore laborious and time-consuming. As a consequence, the cost and price of such a suction valve with individual components can be high, even in high-volume products.

### SUMMARY OF THE INVENTION

In view of the above, an object of the present invention is to provide a novel and improved suction valve as well as fluid control unit with lesser components for flawless utilization in a surgical device, in particular endoscope. The novel suction valve, as well as the fluid control unit, enables uncomplicated assembling and mounting process, hence, lowers the price of high volume product thereof.

In accordance with the present invention, a suction valve with the features of claim 1, a fluid control unit having the features of claim 10, a surgical device with the features of claim 13, a method for producing a suction valve as recited in claim 14 and/or a method for manufacturing a fluid control unit as recited in claim 15 are provided. Advantageous or preferred features of the invention are recited in the dependent claims.

Accordingly, the present invention provides:
- A suction valve for controlling the communication of fluid channels of a surgical device, in particular an endoscope, comprising: a suction chamber; a piston assembly for controlling fluid flow through the suction chamber, wherein the suction chamber and the piston assembly are configured as a single-piece injection-molded workpiece, wherein the suction chamber is formed by a hollow part of the workpiece, which is pulled over the piston assembly.
- A fluid control unit for controlling communication of fluid channels of a surgical device, in particular an endoscope, comprising: a suction valve according to the invention; and a housing assembly accommodating the suction valve and configured to be installed on a casing port of the surgical device.
- A surgical device, in particular endoscope, having a casing port for receiving a fluid control unit according to the invention.
- A method for manufacturing a suction valve, comprising the following steps: injection molding a single-piece workpiece comprising a piston assembly and a hollow part configured to form a suction chamber; and pulling over the hollow part of the workpiece over the piston assembly to form the suction chamber.
- A method for manufacturing a fluid control unit, in particular a fluid control unit according to the invention, comprising the following steps: providing a suction valve according to the invention and/or produced according to a method of the invention; providing a housing assembly configured to be installed on a casing port of a surgical device; and accommodating the suction valve in the housing assembly.

One of the ideas upon which the present invention is based is therefore that fewer and less parts are capable of constructing a suction valve, as well as a fluid control unit, for a surgical device, in particular for an endoscope. The construction of these components obviates thus the necessity of setting up and assembling additional elements, such as seals and springs, which would otherwise be required.

In this way, the present invention provides a suction valve, as well as a fluid control unit, comprising an injection-molded single-piece in a pulled-over configuration set up to provide the fluid control function in an integrated way.

By means of a single-piece injection-molded workpiece design, the suction valve can be easily produced in high quantity by injection molding. Accordingly, a low price per unit is achieved.

Preferably, the single-piece injection-molded workpiece is formed with an elastic material, especially an elastic polymer material.

The merits of injection molding are evident for producing a single-piece workpiece. Various materials can be used, especially injection molding with polymer materials can provide a viable solution by blending and applying various polymers and additives to enhance and achieve the desired features and attributes and can accommodate complex designs. In this way, for example, thermoplastic elastomer materials can be applied. Injection molding is highly efficient, effective, and suitable for high-volume production, simplifying the manufacturing procedure and thus lowering the costs and efforts compared to other molding processes.

Furthermore, advantageously, injection molding allows three-dimensional forming of elastic material, especially elastomeric material, e.g. thermoplastic elastomeric material. In this way, material properties such as resilient elasticity and sealing can be integrated in the single-piece design.

Functional integrity of the suction valve is achieved by the pulled-over configuration of the single-piece injection-molded workpiece. Biased material in the transition between suction chamber and piston assembly can form a resilient section or spring section. In this section, the material in particular is bent or folded, in particular all around, to a predetermined shape. Accordingly, the chamber and the piston formed from one piece are pre-tensioned against each other such that a sealing force is provided which abuts the piston against a sealing surface of the chamber. In this way, the conventionally multiple elements of a suction valve, such as piston, seals, spring, button etc., are integrated in one single-piece injection-molded workpiece forming the suction valve.

Especially, the one-piece design is configured for automated production including automated folding to pull the hollow part over the piston assembly to form the chamber.

The present disclosure therefore also proposes a compact structure of a fluid control unit comprising two separable parts, namely the suction valve and a housing assembly. The fluid control unit is configured for controlling flow connectivity of a surgical device, or rather an endoscope.

The suction valve and the housing assembly can be assembled together as a fluid control unit to perform the fluid control function by accommodating the suction valve in the housing assembly. For mounting the fluid control unit, the one-piece injection-molded suction valve can be simply clipped into the housing assembly to accommodate it. Thereby, the function-integrated configuration of the fluid control unit with essentially only two parts ensures no dislocation or dislodging of conventional elements. Advantageously, the compact structure and one-piece construction of the suction valve according to the present invention simplifies the assembly process of the suction valve with the housing assembly to form the fluid control unit. In this way, the manufacturing time required is reduced.

The present invention therefore provides an advantageous design. The fluid control unit can be formed with only two separate parts with integrated functions: the movable suction valve for switching a fluid connection state, and a housing assembly configured to receive the suction valve and link it to a suction source on one hand and a fluid channel of the surgical instrument on the other. Dividing the construction into distinct but functionally integrated parts allows focusing on the respective functional requirements of elasticity and sealing properties of the suction valve and providing structural integrity and an interface to the surgical instrument of the housing assembly.

Moreover, a fluid control unit with a design according to the invention allowing a single-use layout meets the requirement of providing surgical devices and systems with lower efforts for cleaning and sterilization. Especially, the present invention enables an advantageous solution for sterile single-use parts for surgical instruments, such as endoscopes, wherein the fluid control unit can be a replaceable (single-use) part of the surgical instrument, particularly of a handgrip thereof.

Advantageous designs and further embodiments result from the further dependent claims as well as from the description with reference to the figures in the drawing.

In an embodiment, the piston assembly is configured to have a released state, in which the suction chamber is closed, and a pressed state, in which fluid flow through the suction chamber is possible. The flow connectivity states are switched between the released and the pressed configurations, for example by pushing down and popping up the piston assembly. In particular, the popping up is resilient, such that the valve state can be controlled by pushing the piston assembly. In a further development, the piston assembly is configured to allow intermediate states of partly pushing down the piston for a reduced fluid flow.

In another embodiment, the suction chamber comprises a first axial opening provided on an end-face. In particular, the first axial opening is provided on an axial end face. The first axial opening is configured to communicate with a suction channel of a surgical device. Furthermore, the suction chamber comprises a second radial opening provided on a periphery of the suction chamber, configured to communicate with a suction source. For example, the suction chamber is configured as an essentially hollow cylinder body having an axial opening and a radial opening.

According to an advantageous development, in the release state, the second radial opening is fluidly isolated from the first axial opening, and in the pressed state, the second radial opening is in fluid communication with the first axial opening through the suction chamber.

In accordance with a preferred embodiment, the piston assembly further comprises an elastic deformable button head portion having a button member formed integrated with a spring member. Preferably, the spring member and button head portion are formed in a bending or folding part of the workpiece. Furthermore, the piston assembly comprises a piston member configured as an elongation of the button member. In particular, the piston member protrudes from the button member in an axial direction.

According to an embodiment, the suction chamber is configured as an essentially hollow cylinder body integrated with the piston assembly, in particular under the button head portion. Preferably, the suction chamber also extends axially from the button member in axial direction and surrounds the piston member. Accordingly, the piston member is arranged on the underside of the button member and is extruded into the suction chamber in the axial direction of the cylinder. The piston member moves downwards and upwards with switching between the pressed and released states, together with elastic deformation of the button head portion, especially the spring member of the button head portion.

In a preferred embodiment, the suction chamber comprises at least one vent provided on the periphery of the suction chamber for fluid communication with the outside. Particularly, the at least one vent is provided along a circumference of the button head portion. In this way, fluid communication with the outside is possible in particular in a released state. The suction chamber further comprises an inner lumen inside the suction chamber extending from the at least one vent along the second radial opening to the first axial opening. Through this lumen, openings at a distal end of an endoscope can be in the fluid communication or isolation with the negative pressure source or the atmosphere.

In another preferred embodiment of the suction valve, the suction chamber comprises an inner seat provided on an inner periphery of the suction chamber, wherein the piston member comprises a sealing flange provided at a distal end of the piston member and configured to seal against the inner seat. In particular, the inner seat is a circumferential seat. Alternatively, or in addition, the sealing flange is a circular sealing flange. Preferably, the inner seat and the sealing flange are fluid-tight abutting each other in the released state. In the pressed state, in contrast, a fluid passage is provided between the inner seat and sealing flange.

According to a preferred embodiment of the suction valve, the single-piece injection-molded workpiece further comprises a circumferentially recessed portion provided on the outer periphery of the suction chamber. In this way, the suction valve can be accommodated in a housing assembly in that a receiving portion of the housing assembly engages in the recessed portion of the suction valve. Furthermore, according to an embodiment, a locking member is arranged on the outer periphery of the suction chamber. In particular, the locking member is arranged in or adjacent the recessed portion. The locking member is configured to lock a further degree of freedom in order to avoid movement of the suction valve accommodated in the housing assembly. For example, the locking member can be configured as a rotation lock.

In an embodiment of the fluid control unit, the housing assembly comprises a port member having a through-opening for receiving the outer periphery of the suction chamber of the suction valve in an assembling state, and a tubular coupling member having a first opening arranged on an inner surface of the port member and a second opening arranged on an outer periphery of the coupling member. A suction channel in the coupling member extends from the first opening to the second opening.

According to an embodiment, in an assembly state, the second radial opening of the suction valve is connected to the first opening of the coupling member and in fluid communication with the suction channel of the coupling member. In this way, a vacuum or suction source can be connected via the suction channel to the suction valve such that a fluid flow is controlled by the suction valve.

In the assembly state, the locking member of the suction valve can be form-fitted locked in a notch arranged on the circumference of the housing assembly. This advantageous design can secure that the two assemblies do not come loose and prevents a relative movement thereof. Especially, rotational movement of the suction valve relative to the housing assembly is prevented.

The above embodiments can be combined with each other as desired, if useful. Further possible embodiments, further configurations and implementations of the invention also include combinations, not explicitly mentioned, of features of the invention described herein with respect to the embodiments. In particular, the skilled person will thereby also add individual aspects as improvements or additions to the respective basic form of the present invention.

### BRIEF DESCRIPTION OF THE DRAWINGS

Aspects and advantages of the present invention will be better understood with reference to the following figures accompanied by the detailed description, which emphasize upon clearly illustrating the principles of the present disclosure. In the different figures, the same elements are indicated with the same reference numerals, in which:
- Fig. 1A: is a schematic sectional view of a single-piece injection-molded workpiece for forming a suction valve;
- Fig. 1B: is a schematic sectional view of a suction valve;
- Fig. 2: is a schematic perspective view of a fluid control unit in an unassembled state;
- Fig. 3: is a schematic longitudinal cross-sectional view of a fluid control unit in an assembled state;
- Fig. 4: is a flow diagram of a method for manufacturing a suction valve; and
- Fig. 5: is a flow diagram of a method of manufacturing a fluid control unit.

The accompanying drawings are included to provide a further understanding of the present invention and are incorporated in and constitute a part of this specification. The drawings illustrate particular embodiments of the invention and together with the description serve to explain the principles of the invention. Other embodiments of the invention and many of the attendant advantages of the invention will be readily appreciated as they become better understood with reference to the following detailed description.

It will be appreciated that common and/or well understood elements that may be useful or necessary in a commercially feasible embodiment are not necessarily depicted in order to facilitate a more abstracted view of the embodiments. The elements of the drawings are not necessarily illustrated to scale relative to each other. It will further be appreciated that certain actions and/or steps in an embodiment of a method may be described or depicted in a particular order of occurrences while those skilled in the art will understand that such specificity with respect to sequence is not actually required. It will also be understood that the terms and expressions used in the present specification have the ordinary meaning as is accorded to such terms and expressions with respect to their corresponding respective areas of inquiry and study, except where specific meanings have otherwise been set forth herein.

### DETAILED DESCRIPTION OF EMBODIMENTS

The detailed description includes specific details for providing a thorough understanding of the present invention. However, it will be apparent to those skilled in the art that the various described elements or features may be arranged in various combinations and configurations without departing from the scope of the present invention.

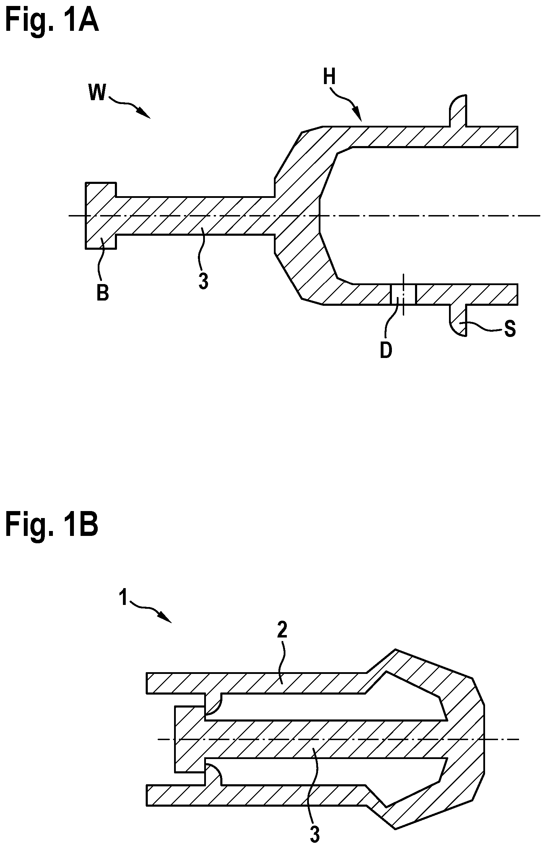

Fig. 1A depicts a schematic sectional view of a single-piece injection-molded workpiece for forming a suction valve. The single-piece injection-molded workpiece W is especially injection molded with an elastic polymer material. The workpiece W can be roughly divided into two parts, namely a hollow part H and a piston assembly 3.

Both parts can share one central axis. For example, the piston assembly 3 has an outer diameter, which is smaller than in inner diameter of the hollow part H. Furthermore, the hollow part H may comprise an opening configured to form a duct D and on an outer diameter a stop member S. The piston assembly 3 may comprise a central part forming a transition to the hollow part H and a longitudinal part extending to a blocking element B at its end.

Fig. 1B depicts a schematic sectional view of a suction valve 1. In this configuration, the single-piece injection-molded workpiece W of Fig. 1A is pulled over to form the suction valve 1. In particular, as shown in Fig. 1B, the hollow part H is pulled over the piston assembly 3 and now configured as a suction chamber 2. This is realized by the inherent characteristics of the injection-molded materials that e.g. possess sufficient elasticity or resilience.

The formerly hollow part H therefore now, after being pulled-over, surrounds the piston assembly 3. Preferably, the stop member S now abuts the blocking element B. In this way, the hollow part forms a suction chamber. The duct D allows application of negative pressure or underpressure to the suction chamber 2 and the piston assembly 3 is resiliently movable with respect to the suction chamber 2.

Thereby, removing the blocking element B from the stop member S opens a fluid communication from the duct D through the suction chamber 2 to an inlet thereof. Accordingly, communication of fluid channels of a surgical device to and from the suction valve can be controlled.

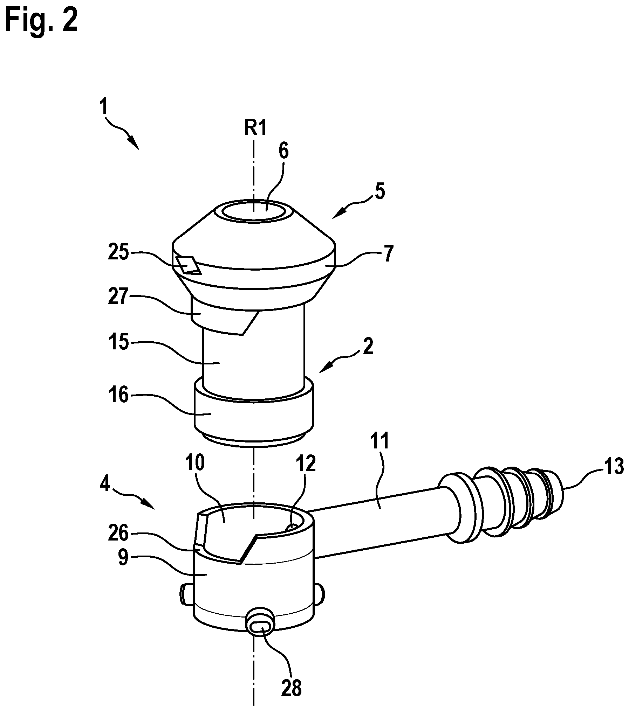

Fig. 2 shows a schematic perspective view of a fluid control unit in an unassembled state.

The unassembled fluid control unit includes two principle components: the suction valve 1 and a housing assembly 4.

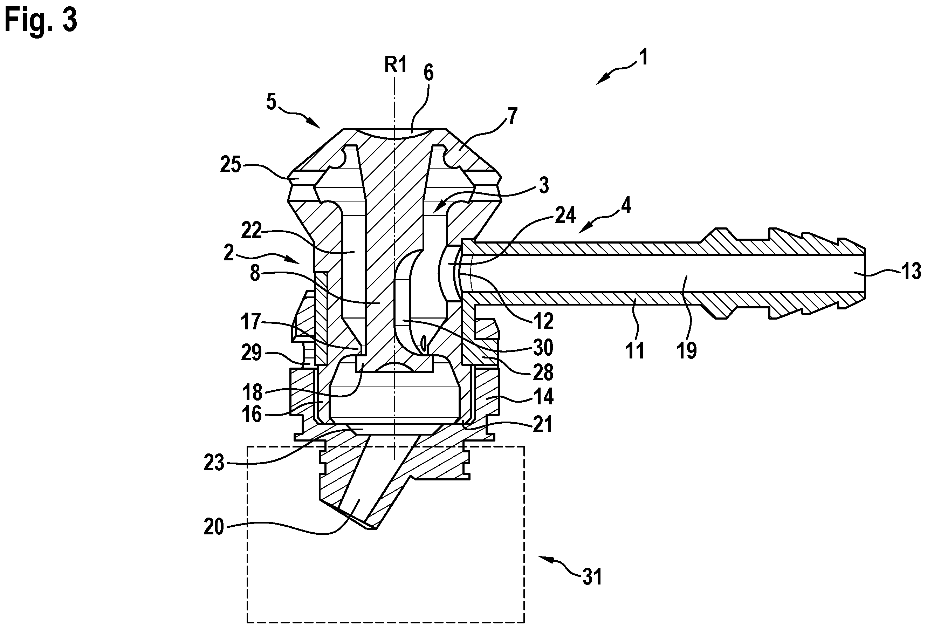

Fig. 3 shows a schematic longitudinal cross-sectional view of a fluid control unit in an assembled state.

As already explained with regard to Fig. 1B, the suction valve 1 has a piston assembly 3 and a suction chamber 2. The piston assembly 3 comprises further an essentially mushroom-shaped button head portion 5 and an axial piston member 8. The suction chamber 2 is configured as an essentially hollow cylinder, which is pulled over the piston assembly 3, in particular attached to the lower side of the button head portion 5.

The button head portion 5 is configured as an integration of a button member 6 and an elastically deformable spring member 7. The button head portion 5, as well as the suction chamber 2, is configured essentially axial symmetric with respect to a longitude axis R1. The piston member 8 is configured as an elongated part protruding from the button head portion 5 in the direction of axis R1. In Fig 3, the piston member 8 is provided on the underside of the button member 6.

The piston assembly 3 has two states, namely a released state and a pressed state. By pressing or releasing the button member 6, switching between the released state and the pressed state can control the fluid communication of the involved fluid channels here the suction channels 19, 20.

When no force is applied to the button member 6, the piston assembly 3 is in its original position after the hollow part is pulled-over and the spring member 7 does not additionally deform, which corresponds to the released state of the piston assembly 3. When the piston assembly 3 is pressed by applying a force to the button member 6, the spring member 7 deforms, which corresponds to the pressed state, and the piston member 8 moves axially relative to the suction chamber 2. Upon the cessation of elastic deformation of the spring member 7, for instance, removal of the applied force, the button head portion 5 is capable of returning or popping up to its initial position of the released state.

On the periphery of the button head portion 5, at least one vent 25 is provided for fluid communication of the inside of the suction valve 1 with the outside, e.g. with the atmosphere. In this way, a reduced fluid flow through a suction channel 19 conected to a suction source is provided also in the released state and the underpressure in the suction chamber 2 can be controlled and limited to a predetermined level. This particularly reduces a resistance for opening the suction valve 2 and avoids the piston member 8 to be sucked on the entry of the suction chamber 2, in particular on an inner seat 17 of the suction chamber 2 forming a stop member.

Referring to Fig. 2, a circumferential blocking ridge 16 can be provided on an outer circumference at the axial end of the suction chamber 2. Furthermore, a circumferential recessed portion 15 extends on the outer periphery of the suction chamber 2. In addition, a locking member 27 can be arranged on the outer periphery of the suction chamber 2, particularly in or adjacent to the recessed portion 15. All these members arranged on the outer circumference serve for coupling the suction valve 1 with the housing assembly 4.

The housing assembly 4 comprises a port member 9 with a through opening 10 for accommodating the suction valve 1, and a tubular coupling member 11 having a distal second opening 13 and a proximal first opening 12 that is arranged on an inner surface of the port member 9. A notch 26 corresponding to the locking member 27 of the suction valve 1 can be provided on the periphery of the port member 9. Beads and ferrules for fluid-tight coupling with fluid channels can be provided on the periphery of the coupling member 11.

In reference to Fig. 2, a longitudinal axis of the through opening 10 can be aligned with the axis R1 when assembling the suction valve 1 with the housing assembly 4. In the assembling process, the suction valve 1 with its axial end enters the through opening 10 of the port member 9 and is moved or slid axially within the through opening 10 along direction of the axis R1 until the blocking ridge 16 is passed through and the locking member 27 engages with the notch 26.

During the assembly, the radial dimension of the blocking ridge 16 can be elastically compressed by the through opening 10 and enlarged upon passing through. In the assembled state shown in Fig. 2, the suction valve is blocked in position by the blocking ridge 16 and the port member 9 of the housing assembly is positioned in the recessed portion 15 of the suction valve 1. The blocking ridge 16 and the button head portion 5 prevent axial loosening or detaching. The locking member 27 engaged in the notch 26 blocks a relative rotation between the suction valve 1 and the housing assembly 4. Accordingly, the suction valve 1 is securely accommodated in the housing assembly 4.

In other embodiments, different elastic deformation may be applied for manufacturing in order to accommodate the suction valve 1 in the housing assembly 4. For example, the through opening 10 can be elastically enlarged by the blocking ridge 16 and upon passing through, the elastic enlargement or deformation of the through opening 10 may disappear again. In other embodiments, the port member 9 may have a slit instead of a notch 26 allowing elastic enlargement of the through opening and/or engaging with a corresponding locking member 27 upon passing through.

The sectional view of Fig. 3 especially illustrates the interior of the assembled fluid control unit in a sectional plane through the longitude axis R1. In the shown state, the suction valve 1 and the housing assembly 4 are entirely mounted on a casing port 14 of a surgical device 31. For example, the port member 9 of the housing assembly 4 has locking members 28 configured to engage with a baffle guide 29 of the casing port 14.

A first axial opening 23 of the suction valve 1 is capable of fluid communication with a distal tip of the surgical device and is arranged on a lower end-face 21 of the suction valve 1. The lower end face 21 is fluid tightly adopted in the casing port 14, which is achieved by a removable fixation between the port member 9 of the housing assembly 4 and the casing port 14, for example by means of a bayonet catch of the locking members 28 and baffle guide 29.

In further embodiments, other types of removable fixation between the port member 9 of the housing assembly 4 are possible, such a threaded connection, or the like.

A second radial opening 24 of the suction chamber 2 is capable of fluid communication with a suction source. The second radial opening 24 is provided on the periphery of the suction valve 1. An inner lumen 22 defined by the inside of the suction valve 1 extends from the vents 25, along the second radial opening 24, and continues to the first axial opening 23. A circumferential sealing flange 18 can be arranged at a distal end of the piston member 8. A circumferential inner seat 17 can be provided on an inner periphery of the suction chamber 2.

The piston member 8 can translate downwards or upwards within the lumen 22 when the button member 6 being pushed down or popped up. When the piston assembly 3 is in the released state, as illustrated in Fig. 3, the sealing flange 18 abuts, in particular from below, the inner seat 17 in a form-fitted and fluid-tight manner. Thus, the lumen 22 can be divided into two fluid-isolated rooms, namely an upper lumen and a lower lumen. The second radial opening 24 may be in fluid communication via the upper lumen as well as the vents 25 with the atmosphere. In the pressed state, a fluid passage or gap can be provided between the inner seat 17 and the sealing flange 18. As a result, the second radial opening 24 can be in fluid communication via the lumen 22 with the first axial opening 23.

Furthermore, as shown in Fig. 3, the piston member 8 may comprise a fluid guiding recess 30 configured to guide the fluid flow in the pressed state from the sealing flange 18 in the most direct way directly through the lumen 22 to the second radial opening 24 and into the first suction channel 19 of the housing assembly.

Accordingly, a first suction channel 19 formed in the coupling member 11 of the housing assembly 4 extends from the second opening 13 to the first opening 12. In the assembled state, the first opening 12 can be form-fitted and fluid-tight connected to the second radial opening 24, through which the first suction channel 19 can be in fluid communication with the lumen 22. A second suction channel 20 in the casing port 14 is in fluid communication with the lumen 22 through the first axial opening 23 on the lower end-face 21 in the mounted state.

In the released state, the fluid communication between the suction channel 19 and the suction channel 20 is cut off. The fluid control unit fluidly isolates the distal tip of a surgical device from the suction source.

In the pressed state, the fluid control unit allows the first suction channel 19 to fluid communicate with the second suction channel 20 through the fluid gap or passage between the sealing flange 18 and the inner seat 17 in the lumen 22. The negative pressure generated by e.g. a suction pump is capable of propagating in the entire fluid mechanism of the surgical instrument for suction.

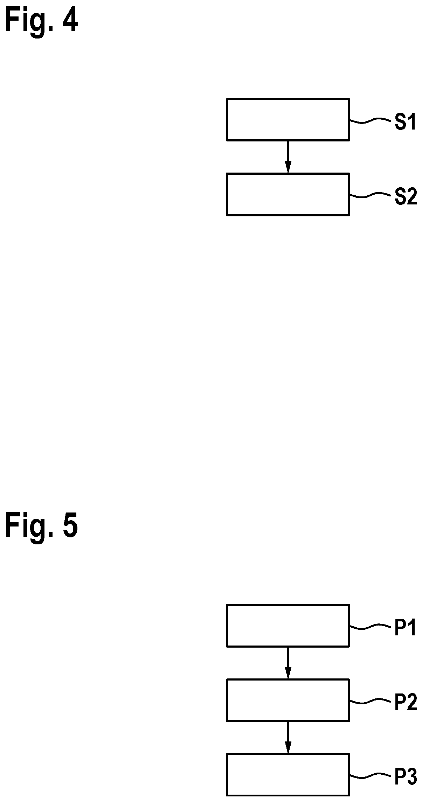

Figure 4 depicts a flow chart that outlines a method for manufacturing a suction valve in accordance with the invention. It comprises:
A step of injection molding S1, wherein a suction chamber 2 and a piston assembly 3 are produced as a single-piece injection-molded workpiece.

The method comprises a further step of pulling over S2, wherein the hollow part of the workpiece is pulled over the piston assembly 3 to form the suction chamber 2.

A flow chart of a manufacturing method for a fluid control unit in accordance with the invention is outlined in Fig. 5.

In a step of providing P1, a suction valve 1 according to the present invention is provided and/or produced.

In a further step of providing P2, a housing assembly 4 configured to be installed on a casing port 14 of a surgical device 31 is provided.

In a step of accommodating P3, the suction valve 1 is received or accommodated in the housing assembly 4.

The assembled fluid control unit can then be mounted on the casing port 14 of the surgical device 31. According to an alternative configuration, the housing assembly
4 can be integrated with the casing port 14, and then the suction valve 1 is mounted within the integral port of the surgical device 31. The surgical device 31 is symbolized in Fig. 3 by a dotted line.

Although specific embodiments of the invention are illustrated and described herein, it will be appreciated by those of ordinary skill in the art that a variety of alternative and/or equivalent implementations exist. It should be appreciated that the exemplary embodiment or exemplary embodiments are examples only and are not intended to limit the scope, applicability, or configuration in any way. Rather, the foregoing summary and detailed description will provide those skilled in the art with a convenient road map for implementing at least one exemplary embodiment, it being understood that various changes may be made in the function and arrangement of elements described in an exemplary embodiment without departing from the scope as set forth in the appended claims and their legal equivalents. Generally, this application is intended to cover any adaptations or variations of the specific embodiments discussed herein.

It will also be appreciated that in this document the terms "comprise", "comprising", "include", "including", "contain", "containing", "have", "having", and any variations thereof, are intended to be understood in an inclusive i.e. non-exclusive sense, such that the process, method, device, apparatus or system described herein is not limited to those features or parts or elements or steps recited but may include other elements, features, parts or steps not expressly listed or inherent to such process, method, article, or apparatus. Furthermore, the terms "a" and "an" used herein are intended to be understood as meaning one or more unless explicitly stated otherwise. Moreover, the terms "first", "second", "third", etc. are used merely as labels, and are not intended to impose numerical requirements on or to establish a certain ranking of importance of their objects.

### LIST OF REFERENCE SIGNS

- 1: suction valve
- 2: suction chamber
- 3: piston assembly
- 4: housing assembly
- 5: button head portion
- 6: button member
- 7: spring member
- 8: piston member
- 9: port member
- 10: through opening
- 11: coupling member
- 12: first opening
- 13: second opening
- 14: casing port
- 15: recessed portion
- 16: blocking ridge
- 17: inner seat
- 18: sealing flange
- 19: suction channel
- 20: suction channel
- 21: end-face
- 22: lumen
- 23: first axial opening
- 24: second radial opening
- 25: vent
- 26: notch
- 27: locking member
- 28: locking member
- 29: baffle guide
- 30: fluid guiding recess
- 31: surgical device
- B: blocking element
- D: duct
- H: hollow part
- S: stop member
- W: workpiece
- S1-S2: Steps
- P1-P3: Steps

## Claims

1. Suction valve (1) for controlling the communication of fluid channels of a surgical device, in particular an endoscope, comprising:
a suction chamber (2);
a piston assembly (3) for controlling fluid flow through the suction chamber (2),
wherein the suction chamber (2) and the piston assembly (3) are configured as a single-piece injection-molded workpiece, wherein the suction chamber (2) is formed by a hollow part of the workpiece, which is pulled over the piston assembly (3).

2. Suction valve (1) according to claim 1,
wherein the piston assembly (3) is configured to have a released state, in which the suction chamber (2) is closed, and a pressed state, in which fluid flow through the suction chamber (2) is possible.

3. Suction valve (1) according to any one of the preceding claims,
wherein the suction chamber (2) comprises
a first axial opening (23) provided on an end-face (21), in particular an axial end face, configured to communicate with a suction channel of a surgical device; and
a second radial opening (24) provided on a periphery of the suction chamber (2), configured to communicate with a suction source.

4. Suction valve (1) according to claim 2 and 3,
wherein in the release state, the second radial opening (24) is fluidly isolated from the first axial opening (23), and in the pressed state, the second radial opening (24) is in fluid communication with the first axial opening (23) through the suction chamber (2).

5. Suction valve (1) according to any one of the preceding claims,
wherein the piston assembly (3) comprises:
an elastic deformable button head portion (5) having a button member (6) formed integrated with a spring member (7);
a piston member (8) configured as an elongation of the button member (6), in particular in an axial direction (R1).

6. Suction valve (1) according to one of the claims 3 to 5,
wherein the suction chamber (2) further comprises:
at least one vent (25) provided on the periphery of the suction chamber (2), particularly along a circumference of the button head portion (5), for fluid communication with the outside, in particular in a released state; and
an inner lumen (22) inside the suction chamber (2) extending from the at least one vent (25) along the second radial opening (24) to the first axial opening(23).

7. Suction valve (1) according to any one of the preceding claims,
wherein the suction chamber (2) comprises an inner seat (17), in particular circumferential seat, provided on an inner periphery of the suction chamber (2),
wherein the piston member (8) comprises a sealing flange (18), in particular circular sealing flange, provided at a distal end of the piston member (8) and configured to seal against the inner seat (17).

8. Suction valve (1) according to claims 2 and 7,
wherein in the released state, the inner seat (17) and the sealing flange (18) are fluid-tight abutting each other; and in the pressed state, a fluid passage is provided between the inner seat (17) and sealing flange (18).

9. Suction valve (1) according to any one of the preceding claims,
wherein the single-piece injection-molded workpiece further comprises a circumferentially recessed portion (15) provided on the outer periphery of the suction chamber (2), and
a locking member (27) arranged on the outer periphery of the suction chamber (2), in particular in or adjacent the recessed portion (15).

10. Fluid control unit for controlling communication of fluid channels of a surgical device, in particular an endoscope, comprising:
a suction valve (1) according to any one of the preceding claims; and
a housing assembly (4) accommodating the suction valve (1) and configured to be installed on a casing port (14) of the surgical device.

11. Fluid control unit according to claim 10,
wherein the housing assembly (4) comprises
a port member (9) having a through-opening (10) for receiving the outer periphery of the suction chamber (2) of the suction valve (1) in an assembling state, and
a tubular coupling member (11) having a first opening (12) arranged on an inner surface of the port member (9) and a second opening (13) arranged on an outer periphery of the coupling member (11); wherein a suction channel (19) in the coupling member (11) extends from the first opening (12) to the second opening (13).

12. Fluid control unit according to claim 11,
wherein the suction valve (1) is configured according to at least claim 3, and
wherein in an assembly state, the second radial opening (24) is connected to the first opening (12) of the coupling member (11) and in fluid communication with the suction channel (19).

13. Surgical device (31), in particular endoscope, having a casing port (14) for receiving a fluid control unit according to any one of the claims 10 to 12.

14. Method for manufacturing a suction valve, in particular a suction valve according to one of the claims 1 to 9, comprising the following steps:
injection molding (S1) a single-piece workpiece comprising a piston assembly (3) and a hollow part configured to form a suction chamber (2); and
pulling over (S2) the hollow part of the workpiece over the piston assembly (3) to form the suction chamber (2).

15. Method for manufacturing a fluid control unit, in particular a fluid control unit according to one of the claims 10 to 12, comprising the following steps:
providing (P1) a suction valve (1) according to one of the claims 1 to 9 and/or producing (P1) a suction valve (1) according to a method of claim 14;
providing (P2) a housing assembly (4) configured to be installed on a casing port (14) of a surgical device; and
accommodating (P3) the suction valve (1) in the housing assembly (4).
